# EUROPEAN PATENT SPECIFICATION

(11) **EP 3 498 245 B1**
(45) Date of publication and mention of the grant of the patent: **07.06.2023**
(21) Application number: 17839515.8
(22) Date of filing: 09.08.2017
(51) Int. Cl.: C09J 7/38, A61F 13/02, A61K 47/32, A61K 47/34, A61K 9/70

(54) **ADHESIVE SKIN PATCH AND WOUND BODY OF ADHESIVE SKIN PATCH**
KLEBENDES HAUTPFLASTER UND GEWUNDENER KÖRPER AUS KLEBENDEM HAUTPFLASTER
TIMBRE ADHÉSIF POUR LA PEAU ET CORPS ENROULÉ DE PATCH ADHÉSIF POUR LA PEAU.

(30) Priority: 09.08.2016 JP 2016156970
(43) Date of publication of application: 19.06.2019
(73) Proprietor: Nitto Denko Corporation, Ibaraki-shi, Osaka 567-8680 (JP)
(72) Inventor: SAKUMA, Takeshi, Ibaraki-shi Osaka 567-8680 (JP); SUZUKI, Mai, Ibaraki-shi Osaka 567-8680 (JP); NAKAMURA, Tetsuya, Ibaraki-shi Osaka 567-8680 (JP); YOSHIDA, Noboru, Ibaraki-shi Osaka 567-8680 (JP)
(74) Representative: Grünecker Patent- und Rechtsanwälte PartG mbB
(86) International application number: PCT/JP2017/028859
(87) International publication number: WO 2018/030447

(56) References cited:
- EP-A1- 2 191 810
- EP-A1- 2 540 289
- EP-A1- 2 921 540
- EP-A2- 1 184 039
- EP-A2- 2 494 995
- WO-A1-2013/180008
- WO-A1-2015/174476
- JP-A- H1 033 655
- JP-A- 2009 273 581
- JP-A- 2011 005 028
- JP-A- 2011 172 831
- JP-A- 2013 169 322
- JP-A- 2014 014 513
- US-A1- 2003 152 612

## Description

### Technical Field

The present invention relates to an adhesive skin patch which is used in a state of being adhered to a skin and a wound body of an adhesive skin patch.

### Background Art

An adhesive skin patch having a base film and a pressure-sensitive adhesive layer provided on one side of the base film is used in a state of being adhered to a skin surface through the pressure-sensitive adhesive layer. When such an adhesive skin patch is adhered to a skin surface, there may be cases where the skin surface gets sweaty and experiences itching, and even suffers irritation. Therefore, adhesive skin patches are required to have high moisture permeability.

Further, because the skin surface has an irregular and complex surface profile, a pressure-sensitive adhesive layer constituting an adhesive skin patch is difficult to completely adhere to the skin surface. In addition, because the skin surface flexibly elongates and shrinks, an adhesive skin patch having good fit feeling as if the adhesive skin patch is not adhered is required, while having good adhesiveness during wear.

### Citation List

### Patent Document

Patent Document 1: JP-A-H10-33655
Patent Document 2: EP 2921540

### Summary of Invention

### Technical Problem

In the light of the above-described present status, the present invention has an object to provide an adhesive skin patch having good adhesiveness and fit feeling to a skin surface, and a wound body of the adhesive skin patch.

### Solution to Problem

As a result of intensive investigations to achieve the above object, the present inventors have found that the above problems can be solved by controlling the thickness of a base film and the thickness of a pressure-sensitive adhesive layer to specified ranges in an adhesive skin patch, and have completed the present invention.

Namely, the present invention relates to an adhesive skin patch including a base film, a pressure-sensitive adhesive layer provided on one side of the base film, and a release liner peelably laminated so as to cover an adhesive surface of the pressure-sensitive adhesive layer,
wherein the pressure-sensitive adhesive layer contains an acrylic copolymer obtained from a monomer mixture containing 40 to 80 mass% of an (meth)acrylic acid alkyl ester monomer and 10 to 60 mass% of an alkoxy group-containing ethylenically unsaturated monomer, and
wherein the base film has a thickness of 1 to 9 µm, and the pressure-sensitive adhesive layer has a thickness of 16 to 50 µm.

### Advantageous Effects of the Invention

According to the present invention, the adhesive skin patch of the present invention has good adhesiveness to a skin surface and good fit feeling as if the adhesive skin patch is not adhered.

### Description of Embodiments

### (Base film)

The base film constituting the adhesive skin patch of the present invention is required to have a thickness of 1 to 9 µm, and the thickness thereof is preferably 3 to 9 µm. When the thickness of the base film is less than 1 µm, there is a possibility that the adhesive skin patch is broken during the use of the adhesive skin patch or peeling away from the skin surface after use. On the other hand, when the thickness of the adhesive skin patch exceeds 9 µm, a feeling of strangeness such as stretched feeling is sometimes generated to flexible movement of a skin surface during wearing the adhesive skin patch to the skin surface and a catch is sometimes generated at the edge of the adhesive skin patch depending on the kind of the base film.

The base film can be obtained from materials such as a urethane polymer such as polyether urethane or polyester urethane, an amide polymer such as polyether polyamide block polymer, an acrylic polymer such as polyacrylate, an olefinic polymer such as polyethylene, polypropylene or ethylene-vinyl alcohol copolymer, and a polyester polymer such as polyether polyester. Those base films are preferably selected from a material having water vapor permeability in order to prevent stuffiness and whitening when adhering to a skin surface. For example, urethane-based and amide-based films are preferably used.

The base film may include any one of the above-described materials and may be a laminate film obtained by laminating a plurality of films including optional materials. The base film can be laminated with a cloth such as a woven fabric, a non-woven fabric, a knitted fabric or a net.

### (Pressure-sensitive adhesive layer)

In the present invention, the pressure-sensitive adhesive layer has a thickness of 16 to 50 µm and preferably 20 to 35 µm. When the thickness of the pressure-sensitive layer is less than 16 µm, adhesiveness to the skin is not sufficient and there is a possibility that the edge of the adhesive skin patch is easy to be peeled as the case may be. On the other hand, when the thickness of the pressure-sensitive adhesive layer exceeds 50 µm, there are possibilities that the edge of the adhesive skin patch is caught, the pressure-sensitive adhesive layer is easy to protrude from the edge and the edge is contaminated, during the adhesive skin patch being adhered to the skin surface.

The pressure-sensitive adhesive layer contains an acrylic copolymer for the reasons that a skin adhesive force is easily adjusted and skin irritation is less when the pressure-sensitive adhesive layer has been brought into contact with a skin.

The acrylic copolymer is an acrylic copolymer obtained from a monomer mixture containing a (meth)acrylic acid alkyl ester monomer and an alkoxy group-containing ethylenically unsaturated monomer.

The (meth)acrylic acid alkyl ester monomer is a main component for imparting good skin adhesiveness to the pressure-sensitive adhesive layer, and it is particularly effective to use a long chain alkyl group having 6 or more, preferably 6 to 18, carbon atoms in the alkyl moiety. The (meth)acrylic acid alkyl ester monomer has advantages that irritation to a skin is relatively weak and decrease of the adhesive force is difficult to occur even by long-term use.

Specific examples of the (meth)acrylic acid alkyl ester monomer include butyl ester, propyl ester, octyl ester, nonyl ester, decyl ester, dodecyl ester and lauryl ester of acrylic acid or methacrylic acid. Those can be used in alone or as mixtures of two or more kinds. Needless to say, an alkyl ester chain of those may be a straight chain and may be a branched chain.

The alkoxy group-containing ethylenically unsaturated monomer is a component for imparting water vapor permeability, a so-called moisture permeability, to the acrylic copolymer. The alkoxy group-containing ethylenically unsaturated monomer is not particularly limited, but, for example, alkoxyalkyl acrylates having an alkoxy group having 1 to 4 carbon atoms, such as methoxypolyethylene glycol acrylate, ethoxydiethylene glycol acrylate, butoxydiethylene glycol acrylate, methoxyethyl acrylate, ethoxyethyl acrylate and butoxyethyl acrylate are preferably used.

The acrylic copolymer is an acrylic copolymer obtained by copolymerization of the (meth)acrylic acid alkyl ester and the alkoxy group-containing ethylenically unsaturated monomer and more preferably an acrylic copolymer obtained by copolymerization of the (meth)acrylic acid alkyl ester, the alkoxy group-containing ethylenically unsaturated monomer and the carboxyl group-containing ethylenically unsaturated monomer.

In the acrylic copolymer of the present invention, as necessary, in addition to the above-mentioned respective monomers, a monomer such as styrene, vinyl acetate or N-vinyl-2-pyrrolidone may be appropriately copolymerized as a modifier resin for performing various modifications such as incorporation of hydrophilicity.

The content of the (meth)acrylic acid alkyl ester monomer in the acrylic copolymer is a range of 40 to 80 mass% and preferably 50 to 75 mass%. When the content of the (meth)acrylic acid alkyl ester monomer is in the above-mentioned range, the pressure-sensitive adhesive layer obtained shows good adhesiveness to a skin, does not generate the phenomenon that an adhesive remains on the skin surface when peeling away from the skin, and has excellent peelability.

The content of the alkoxy group-containing ethylenically unsaturated monomer in the acrylic copolymer is a range of 10 to 60 mass%, preferably 20 to 60 mass% and still more preferably 25 to 50 mass%. When the content of the alkoxy group-containing ethylenically unsaturated monomer is in the above range, the resulting copolymer can impart excellent moisture permeability to the pressure-sensitive adhesive layer.

When the carboxyl group-containing ethylenically unsaturated monomer is further contained, the content of the alkoxy group-containing ethylenically unsaturated monomer is a range of preferably 10 to 50 mass% and more preferably 20 to 45 mass%.

The content of the carboxyl group-containing ethylenically unsaturated monomer is preferably 1 to 10 mass% and more preferably 3 to 8 mass%. When the content of the carboxyl group-containing ethylenically unsaturated monomer is in the above range, the resulting copolymer can impart excellent cohesive force to the pressure-sensitive adhesive layer, can suppress the phenomenon that an adhesive residue remains on the skin surface and additionally can suppress skin irritation.

Weight average molecular weight of a soluble portion in a solvent for molecular weight measurement of the acrylic copolymer is desirably adjusted to 500,000 to 2,500,000 and preferably about 600,000 to 2,000,000.

The weight average molecular weight and molecular weight distribution used herein are values measured using gel permeation chromatography (GPC). A sample for measurement is dissolved in tetrahydrofuran, a soluble portion passing through a 0.45 µmφ membrane filter is subjected to GPC measurement and the value is calculated in terms of polystyrene.

The pressure-sensitive adhesive layer of the present invention may contain a liquid or pasty component at room temperature (25°C). The liquid or pasty component at room temperature is a component for imparting flexibility to the pressure-sensitive adhesive layer and reducing skin irritation when the pressure-sensitive adhesive layer has been brought into contact with the skin.

The liquid or pasty component at room temperature is not particularly limited so long as the component is compatible with the acrylic copolymer. Examples of the component that can be used include esters of phthalic acid, maleic acid, adipic acid, stearic acid or various fatty acids and alkyl alcohols, and esters of those acids and polyhydric alcohols such as ethylene glycol and glycerin. More specifically, examples of a monohydric alcohol ester include dibutyl phthalate, di-2-ethylhexyl phthalate, dibutyl adipate, di-2-ethylhexyl sebacate, dibutyl maleate, ethyl myristate, isopropyl myristate, isopropyl palmitate, butyl stearate, isopropyl isostearate, hexyl laurate, cetyl lactate, myristyl lactate, diethyl phthalate, octyldodecyl myristate, octyldodecyl oleate, hexyldecyl dimethyloctanoate, cetyl 2-ethylhexanoate, isocetyl 2-ethylhexanoate, stearyl 2-ethylhexanoate and dioctyl succinate. Examples of a dihydric or higher alcohol ester include propylene glycol dicaprylate, propylene glycol dicaprate, propylene glycol diisostearate, glyceryl monocaprylate, glyceryl tricaprylate, glyceryl tri-2-ethylhexanoate, glyceryl tricaprate, glyceryl trilaurate, glycery triisostearate, glycery trioleate and trimethylolpropane tri-2-ethylhexanoate. Those esters can be used one kind or as mixtures of two or more kinds. The liquid or pasty component at room temperature can be appropriately determined according to other factors in the adhesive skin patch, but from the standpoint of compatibility with the acrylic copolymer, carboxylic acid ester is preferably used, glycerin fatty acid ester is more preferably used and glycerin ester of a saturated fatty acid is still more preferably used.

Examples of the fatty acid include saturated fatty acids such as caprylic acid, capric acid and 2-ethylhexanoic acid. Those acids can be can be used alone or as combinations of two or more thereof. Those saturated fatty acids do not have an unsaturated double bond and therefore can prevent oxidative deterioration of the pressure-sensitive adhesive, that is, the pressure-sensitive adhesive layer.

When the above-described caprylic acid and the like are used as saturated fatty acids, glyceryl tricaprylate, glyceryl tricaprate and glyceryl tri-2-ethylhexanoate are obtained as esters of various saturated fatty acids and alcohols. Of those carboxylic acid esters, glyceryl tricaprylate is particularly preferably used from the standpoint of compatibility with the acrylic copolymer, and the like.

The content ratio between the acrylic copolymer and the liquid or pasty component at room temperature such as the carboxylic acid ester is, for example, such that the content ratio of the liquid or pasty component at room temperature is preferably 20 to 100 parts by mass per 100 parts by mass of the acrylic copolymer. When the content ratio of the component is in the above range, sufficient flexibility can imparted to the pressure-sensitive adhesive layer. As a result, the pressure-sensitive adhesive layer has good adhesiveness to a skin, suppresses physical skin irritation when peeling the patch from the skin and can achieve good peelability.

The pressure-sensitive adhesive layer constituting the adhesive skin patch is preferably subjected to a crosslinking treatment in order to impart appropriate cohesive force to the pressure-sensitive adhesive layer. The crosslinking treatment includes physical crosslinking by irradiation with ionizing radiation such as electron beams, γ rays or X rays, and chemical crosslinking by a crosslinking agent. The pressure-sensitive adhesive is preferably subjected to the chemical crosslinking treatment by a crosslinking agent from the standpoints of handling properties and good reproducibility of crosslinking. For example, a crosslinking agent is added to a pressure-sensitive adhesive composition having a material constituting a pressure-sensitive adhesive layer added thereto, and the resulting pressure-sensitive adhesive composition is heat-treated to perform a crosslinking treatment. Examples of such a crosslinking agent include crosslinking agents used in conducting a crosslinking treatment, such as an isocyanate crosslinking agent, a peroxide crosslinking agent or a metal chelate crosslinking agent.

The pressure-sensitive adhesive layer having been subjected to the crosslinking treatment as above holds a balance between appropriate adhesiveness to a skin and cohesive force by the degree of crosslinking, but a molecular weight and molecular weight distribution of the acrylic copolymer also affect the adjustment of those.

In the adhesive skin patch of the present invention, the surface of the pressure-adhesive layer is covered with a release liner until using the adhesive skin patch for the purpose of preventing the surface of the pressure-sensitive adhesive layer from being contaminated. As the release liner, there may be used release liners generally used for skin and pressure-sensitive adhesive tapes adhering to a skin. Specifically, a release liner obtained by coating the surface of wood free paper, glassine paper, parchment paper or the like with a release agent having release performance such as a silicone resin or a fluorine resin, a release liner obtained by anchor-coating wood free paper with a resin or a release paper obtained by coating the surface of a polyethylene laminate or the like with a release agent having release performance such as a silicone resin or a fluorine resin can be used.

Although not particularly shown in the drawing, in the adhesive skin patch of the present invention, a supporting film can be peelably laminated on the surface opposite the surface having the pressure-sensitive adhesive layer formed thereon, of the base film.

Preferred examples of the material for the supporting film include plastic films (for example, polyethylene, polypropylene, polyester and laminated composites of those) and papers (for example, wood free paper and craft paper). Of those materials, a plastic film having transparency can be adhered while seeing an adhering region through an adhesive skin patch, and therefore has particularly remarkable usefulness when fixing a medical instrument such as catheter. To peelably adhere the supporting film to the back of the base film, the conventional method such as inflation molding, extrusion laminate molding, lamination molding or casting can be used.

The thickness of the supporting film varies depending on the material thereof, but is generally preferably about 15 to 200 µm and more preferably about 20 to 100 µm.

Although not shown in the drawing, the adhesive skin patch of the present invention can be wound in the state of being laminated on the supporting film as described above to form a wound body.

One example of a method for manufacturing the adhesive skin patch of the present invention is described below, but the invention should not be construed as being limited to those.

A monomer mixture containing, for example, a (meth)acrylic acid alkyl ester monomer, an alkoxy group-containing ethylenically unsaturated monomer and as necessary, a carboxyl group-containing ethylenically unsaturated monomer is subjected to a copolymerization reaction by the general radical polymerization in an organic solvent to prepare an acrylic copolymer solution. Next, 20 to 100 parts by mass of a liquid or pasty component at room temperature such as carboxylic acid ester and as necessary, a crosslinking agent are added to 100 parts by mass of a solid portion (acrylic copolymer) of the acrylic copolymer solution to prepare a solution of a pressure-adhesive composition.

The pressure-sensitive adhesive solution thus obtained is applied to the entire surface of the release-treated surface of a release liner or is partially applied to the surface thereof. When the pressure-sensitive adhesive solution is partially applied, the pressure-sensitive adhesive solution is preferably applied in the form of, for example, dots or steaks. In the case of applying the solution in the form of steaks, the application includes linear application and wavy application. However, the solution may be applied in any form so long as spaces between steaks are secured. The wavy application is preferred from that the change with the passage of time of the cross-sectional area between spaces is small during adhering to a skin. After drying the pressure-sensitive adhesive solution applied to the release liner, a base film having a supporting film laminated thereon is adhered to the release liner to obtain an adhesive skin patch.

### Examples

The present invention is described in more detail below by reference to examples, but those examples should not be construed as limiting the present invention. All "parts" and "%" in the following examples mean "parts by mass" and "mass%", respectively.

### (Example 1)

A solution of an acrylic copolymer was obtained by copolymerization reaction of a monomer mixture containing 65 parts of isononyl acrylate, 30 parts of 2-methoxyethyl acrylate and 5 parts of acrylic acid. Then, 60 parts of glyceryl tricaprylate and 0.04 parts of a trifunctional isocyanate compound as a crosslinking agent with respect to 100 parts of the acrylic copolymer were added to the solution of the acrylic copolymer to prepare a homogeneous pressure-sensitive adhesive solution.

The solution obtained was then applied to the release-treated surface of a release liner, one side of which having been subjected to a silicone release treatment, such that the thickness after drying became 26 µm, and then dried to form a pressure-sensitive adhesive layer. The pressure-sensitive adhesive layer was adhered to a polyurethane film surface of a laminate film obtained by peelably laminating a 40 µm thick polypropylene film (supporting film) on a 8 µm thick polyurethane film (base film). Thus, an adhesive skin patch of Example 1 was obtained.

### (Examples 2 to 4 and Comparative Examples 1 to 4)

Adhesive skin patches of Examples 2 to 4 and Comparative Examples 1 to 4 were obtained in the same manner as in Example 1, except that the kind of the base film, the thickness of the base and the thickness of the pressure-sensitive adhesive layer after drying were changed as shown in Table 1.

The details of the adhesive skin patches of Examples 1 to 4 and Comparative Examples 1 to 4 are shown in Table 1.

**[Table 1]**

| | Kind of base film | Thickness of base (µm) | Thickness of pressure-sensitive adhesive layer (µm) |
|---|---|---|---|
| Example 1 | PU | 8 | 26 |
| Example 2 | PET | 6 | 26 |
| Example 3 | PET | 6 | 16 |
| Example 4 | PET | 6 | 45 |
| Comparative Example 1 | PU | 30 | 30 |
| Comparative Example 2 | PET | 6 | 11 |
| Comparative Example 3 | PET | 6 | 65 |
| Comparative Example 4 | PET | 25 | 26 |

| | | | |
|---|---|---|---|
| PU: Polyurethane film, PET: Polyethylene terephthalate film | | | |

### (Functional evaluation during wear)

Each of the adhesive skin patches of Examples 1 to 4 and Comparative Examples 1 to 4 was cut into a rectangular shape of 50 mm wide and 50 mm long. A release liner was peeled away from each adhesive skin patch cut, and a pressure-sensitive adhesive layer surface of the adhesive skin patch was adhered to a forearm portion of five volunteers. A supporting film was peeled away, and the adhesive skin patch was allowed to stand at room temperature in this state. After the lapse of 3 hours, stretched feeling of the adhered region, lifting state of the edge of the adhesive skin patch and catch at the edge of the adhesive skin patch were evaluated in 3 grades.

**[Table 2]**

| | Stretched feeling | Lifting state | Catch |
|---|---|---|---|
| Example 1 | 3 | 3 | 3 |
| Example 2 | 3 | 3 | 3 |
| Example 3 | 3 | 3 | 3 |
| Example 4 | 3 | 3 | 3 |
| Comparative Example 1 | 1 | 3 | 3 |
| Comparative Example 2 | 3 | 1 | 3 |
| Comparative Example 3 | 3 | 3 | 1 |
| Comparative Example 4 | 1 | 3 | 1 |

### (Stretched feeling)

3: No stretched feeling
2: Slight stretched feeling
1: Stretched feeling

### (Lifting state)

3: No lifting
2: Slight lifting at edge
1: Lifting of a half or more

### (Catch)

3: No catch at edge
2: Slight catch at edge
1: Catch at edge

The results are shown in Table 2.

The adhesive skin patches of Examples 1 to 4 did not generate lifting at the edge and showed satisfactory adhesiveness to a skin, during wear. Furthermore, the adhesive skin patches of Examples 1 to 4 showed satisfactory follow-up properties to the movement of a skin, did not substantially have stretched feeling and had satisfactory fit feeling as if the adhesive skin patch is not adhered. Additionally, the adhesive skin patches of Examples 1 to 4 did not generate catch at the edge during wear and did not generate contamination by protrusion of the pressure-sensitive adhesive layer.

On the other hand, the adhesive skin patches of Comparative Examples 1 and 4 had stretched feeling during wear and had the adhering feeling. Above all, the adhesive skin patch of Comparative Example 4 generated catch at the edge during wear. The adhesive skin patch of Comparative Example 2 generated lifting at the edge during wear and had poor adhesiveness to a skin. The adhesive skin patch of Comparative Example 3 generated catch at the edge during wear and contamination by protrusion of the pressure-sensitive adhesive layer was slightly confirmed.

### (Example 5)

A solution of an acrylic copolymer was obtained by copolymerization reaction of a monomer mixture containing 65 parts of isononyl acrylate, 30 parts of 2-methoxyethyl acrylate and 5 parts of acrylic acid. Then, 60 parts of glyceryl tricaprylate and 0.13 parts of a trifunctional isocyanate compound as a crosslinking agent with respect to 100 parts of the acrylic copolymer were added to the solution of the acrylic copolymer to prepare a homogeneous pressure-sensitive adhesive solution.

The solution thus obtained was then applied to the release-treated surface of a release liner, one side of which having been subjected to a silicone release treatment, such that the thickness after drying became 16 µm, and then dried to form a pressure-sensitive adhesive layer. The pressure-sensitive adhesive layer was adhered to a polyurethane film surface of a laminate film obtained by peelably laminating a 40 µm thick polypropylene film (supporting film) on a 8 µm thick polyurethane film (base film). Thus, an adhesive skin patch of Example 5 was obtained.

### (Examples 6 to 9 and Comparative Examples 5 to 8)

Adhesive skin patches of Examples 6 to 9 and Comparative Examples 5 to 8 were obtained in the same manner as in Example 5, except that the thickness of the polyurethane film and the thickness of the pressure-sensitive adhesive layer after drying were changed as shown in Table 3.

The details of the adhesive skin patches of Examples 5 to 9 and Comparative Examples 5 to 8 are shown in Table 3.

**[Table 3]**

| | Thickness of base (µm) | Thickness of pressure-sensitive adhesive layer (µm) |
|---|---|---|
| Example 5 | 8 | 16 |
| Example 6 | 2 | 23 |
| Example 7 | 5 | 23 |
| Example 8 | 8 | 23 |
| Example 9 | 8 | 46 |
| Comparative Example 5 | 13 | 23 |
| Comparative Example 6 | 30 | 23 |
| Comparative Example 7 | 8 | 10 |
| Comparative Example 8 | 8 | 69 |

### (Modulus)

Each of the adhesive skin patches of Examples 5 to 9 and Comparative Examples 5 to 8 was cut into a rectangular shape of 10 mm wide and 100 mm long. A release liner was peeled away from each patch and then mounted such that chuck distance of a tensile tester is 40 mm. A supporting film was peeled away from the patch and the patch was stretched in a rate of 300 mm/min. Loads (N/10 mm) when the degree of elongation reached 10%, 30% and 100% were read (average value of n=3).

### (Moisture permeability)

10 mL of purified water was put into a 38 mm diameter glass vessel and the mouth of the glass vessel was covered with each of the adhesive skin patches of Examples 5 to 9 and Comparative Examples 5 to 8 from which the release liner and the supporting film had been peeled away, and the glass vessel was sealed by winding a pressure-sensitive adhesive tape around the periphery of the mouth portion of the glass vessel. After measuring the weight of the glass vessel, the glass vessel was stored under the conditions of a temperature of 40°C and a humidity of 30% RH for 24 hours. After storage for 24 hours, the weight of the glass vessel was measured, and the amount of water vapor passed through the adhesive skin patch was obtained from the difference in weight between the weight before storage and the weight after storage. Moisture permeation amount (moisture permeability) per 1 m² of the test piece was calculated from the value obtained (average value of n=3).

### (Pressure-sensitive adhesive force)

The supporting film was peeled away from each of the adhesive skin patches of Examples 5 to 9 and Comparative Examples 5 to 8, and a pressure-sensitive adhesive tape (No. 31B: manufactured by Nitto Denko Corporation) was adhered to the polyurethane film surface of the patch. The patch was cut into a rectangular shape of 10 mm wide and about 70 mm long. After peeling away the release liner from the patch, the pressure-adhesive layer surface of the patch was adhered to a bakelite plate and the resulting laminate was press-bonded by reciprocating a roller having a weight of 2 kg once. After the lapse of 30 minutes, the patch was peeled away at a peel angle of 180° in a rate of 300 mm/min by a tensile tester, and the peel force in the peeling was measured (average number of n=3).

**[Table 4]**

| | Modulus [N/10 mm] | | | Moisture permeability [g/cm²·24h] | Adhesive force [N/10 mm] |
|---|---|---|---|---|---|
| | 10% | 30% | 100% | | |
| Example 5 | 0.07 | 0.24 | 0.49 | 1422 | 3.8 |
| Example 6 | 0.04 | 0.09 | 0.18 | 1533 | 3.4 |
| Example 7 | 0.08 | 0.18 | 0.33 | 1434 | 2.7 |
| Example 8 | 0.10 | 0.27 | 0.52 | 1265 | 3.1 |
| Example 9 | 0.12 | 0.31 | 0.58 | 817 | 4.3 |
| Comparative Example 5 | 0.19 | 0.57 | 1.19 | 1210 | 2.6 |
| Comparative Example 6 | 0.23 | 0.65 | 1.29 | 995 | 2.5 |
| Comparative Example 7 | 0.05 | 0.24 | 0.51 | 2224 | 2.1 |
| Comparative Example 8 | 0.10 | 0.29 | 0.58 | 742 | 4.3 |

The results are shown in Table 4.

The adhesive skin patches of Examples 5 to 9 have appropriate adhesive force and therefore show satisfactory adhesiveness to a skin surface. Furthermore, the patches have low modulus and high moisture permeability and as a result, are difficult to feel stuffiness and stretched feeling during wear, and showed satisfactory fit feeling as if the adhesive skin patch is not adhered.

On the other hand, the adhesive skin patches of Comparative Examples 5 and 6 had the tendency of high modulus as compared with the Examples, had stretched feeling during wear and had apparent feeling as if the adhesive skin patch is adhered. The adhesive skin patch of Comparative Example 7 had low modulus and high moisture permeability, but had weak adhesive force and was easy to be peeled during wear. The adhesive skin patch of Comparative Example 8 had low level of moisture permeability, was easy to generate stuffiness and had a possibility of itch sensation.

## Claims

1. An adhesive skin patch comprising a base film, a pressure-sensitive adhesive layer provided on one side of the base film, and a release liner peelably laminated so as to cover an adhesive surface of the pressure-sensitive adhesive layer,
wherein the pressure-sensitive adhesive layer contains an acrylic copolymer obtained from a monomer mixture containing 40 to 80 mass% of an (meth)acrylic acid alkyl ester monomer and 10 to 60 mass% of an alkoxy group-containing ethylenically unsaturated monomer, and
wherein the base film has a thickness of 1 to 9 µm, and the pressure-sensitive adhesive layer has a thickness of 16 to 50 µm.

2. The adhesive skin patch according to claim 1, wherein the base film is at least one selected from the group consisting of a urethane polymer, an amide polymer, an acrylic polymer, an olefinic polymer and an ester polymer.

3. The adhesive skin patch according to claim 1 or 2, wherein the acrylic copolymer contains an acrylic copolymer obtained from the monomer mixture further containing a carboxy group-containing ethylenically unsaturated monomer.

4. The adhesive skin patch according to claim 1 to 3, wherein the acrylic copolymer contains an acrylic copolymer obtained from a monomer mixture containing 40 to 80 mass% of the (meth)acrylic acid alkyl ester monomer, 10 to 50 mass% of the alkoxy group-containing ethylenically unsaturated monomer and 1 to 10 mass% of the carboxyl group-containing ethylenically unsaturated monomer.

5. The adhesive skin patch according to any one of claims 1 to 4, which further comprises a supporting film peelably laminated so as to cover another side of the base film.

6. A wound body of an adhesive skin patch, obtained by winding the adhesive skin patch according to claim 5.

## Patentansprüche

1. Hautklebepflaster, umfassend einen Basisfilm, eine druckempfindliche Klebeschicht, die auf einer Seite des Basisfilms bereitgestellt ist, und eine Trennschicht, die abziehbar so laminiert ist, dass sie eine Klebeoberfläche der druckempfindlichen Klebeschicht bedeckt,
wobei die druckempfindliche Klebeschicht ein Acryl-Copolymer enthält, erhalten aus einer Monomermischung enthaltend 40 bis 80 Masse-% eines (Meth)acrylsäure-Alkylester-Monomers und 10 bis 60 Masse-% eines Alkoxygruppen-enthaltenden ethylenisch ungesättigten Monomers, und
wobei der Basisfilm eine Dicke von 1 bis 9 µm hat und die druckempfindliche Klebeschicht eine Dicke von 16 bis 50 µm hat.

2. Hautklebepflaster nach Anspruch 1, wobei der Basisfilm mindestens einer ausgewählt aus der Gruppe bestehend aus einem Urethanpolymer, einem Amidpolymer, einem Acrylpolymer, einem olefinischen Polymer und einem Esterpolymer ist.

3. Hautklebepflaster nach Anspruch 1 oder 2, wobei das Acryl-Copolymer ein Acryl-Copolymer enthält, erhalten aus der Monomermischung ferner enthaltend ein Carboxylgruppenenthaltendes ethylenisch ungesättigtes Monomer.

4. Hautklebepflaster nach einem der Ansprüche 1 bis 3, wobei das Acryl-Copolymer ein Acryl-Copolymer enthält, erhalten aus einer Monomermischung enthaltend 40 bis 80 Masse-% des (Meth)acrylsäurealkylester-Monomers, 10 bis 50 Masse-% des Alkoxygruppen-enthaltenden ethylenisch ungesättigten Monomers und 1 bis 10 Masse-% des Carboxylgruppen-enthaltenden ethylenisch ungesättigten Monomers.

5. Hautklebepflaster nach einem der Ansprüche 1 bis 4, das ferner einen Trägerfilm umfasst, der abziehbar so laminiert ist, dass er eine andere Seite des Basisfilms bedeckt.

6. Wundkörper eines Hautklebepflaster, erhalten durch Aufwickeln des Hautklebepflasters nach Anspruch 5.

## Revendications

1. Timbre dermique adhésif comprenant un film de base, une couche adhésive sensible à la pression prévue sur un côté du film de base, et une pellicule de protection stratifiée de manière pelable de manière à recouvrir une surface adhésive de la couche adhésive sensible à la pression,
dans lequel la couche adhésive sensible à la pression contient un copolymère acrylique obtenu d'un mélange de monomère contenant 40 à 80 % en masse d'un monomère d'alkylester d'acide (méth)acrylique et 10 à 60 % en masse d'un monomère éthyléniquement insaturé contenant un groupe alcoxy, et
dans lequel le film de base présente une épaisseur de 1 à 9 µm, et la couche adhésive sensible à la pression présente une épaisseur de 16 à 50 µm.

2. Timbre dermique adhésif selon la revendication 1, dans lequel le film de base est au moins un sélectionné dans le groupe constitué par un polymère uréthane, un polymère amide, un polymère acrylique, un polymère oléfinique et un polymère ester.

3. Timbre dermique adhésif selon la revendication 1 ou 2, dans lequel le copolymère acrylique contient un copolymère acrylique obtenu du mélange de monomère contenant en outre un monomère éthyléniquement insaturé contenant un groupe carboxy.

4. Timbre dermique adhésif selon la revendication 1 à 3, dans lequel le copolymère acrylique contient un copolymère acrylique obtenu d'un mélange de monomère contenant 40 à 80 % en masse du monomère d'alkylester d'acide (méth)acrylique, 10 à 50 % en masse du monomère éthyléniquement insaturé contenant un groupe alcoxy et 1 à 10 % en masse du monomère éthyléniquement insaturé contenant un groupe carboxyle.

5. Timbre dermique adhésif selon l'une quelconque des revendications 1 à 4, qui comprend en outre un film de support stratifié de manière pelable de manière à recouvrir un autre côté du film de base.

6. Corps enroulé d'un timbre dermique adhésif, obtenu par enroulement du timbre dermique adhésif selon la revendication 5.
